# EUROPÄISCHE PATENTSCHRIFT

(11) **EP 3 010 443 B1**
(45) Veröffentlichungstag und Bekanntmachung des Hinweises auf die Patenterteilung: **14.09.2022**
(21) Anmeldenummer: 14731260.7
(22) Anmeldetag: 18.06.2014
(51) Int. Cl.: A61F 2/07, A61F 2/97, A61F 2/89

(54) **STENTGRAFT**
STENT GRAFT
ENDOPROTHÈSE

(30) Priorität: 20.06.2013 DE 102013106463
(43) Veröffentlichungstag der Anmeldung: 27.04.2016
(73) Patentinhaber: JOTEC GmbH, 72379 Hechingen (DE)
(72) Erfinder: LESMEISTER, Rainer, 72827 Wannweil (DE); BARTHOLD, Franz-Peter, 72336 Balingen (DE)
(74) Vertreter: Witte, Weller & Partner Patentanwälte mbB
(86) Internationale Anmeldenummer: PCT/EP2014/062784
(87) Internationale Veröffentlichungsnummer: WO 2014/202645

(56) Entgegenhaltungen:
- US-A1- 2010 152 711
- US-A1- 2012 130 475

## Beschreibung

Die vorliegende Erfindung betrifft einen Stentgraft, mit einem hohlzylindrischen Körper und einem Hüllkatheter, wobei der Körper zumindest einen selbstexpandierenden Stent und ein Prothesenmaterial aufweist, wobei der Körper in einem komprimierten Zustand einen Umfang aufweist, der kleiner ist als ein Umfang des Körpers in einem expandierten Zustand.

Derartige Stentgrafts sind im Stand der Technik hinreichend bekannt. Es handelt sich um ein medizinisches Implantat, welches in Hohlorgane, bspw. Blutgefäße, eines Patienten eingebracht wird, um die dort aufgrund Krankheiten auftretenden Verengungen offenzuhalten und dadurch den Blutstrom aufrechtzuerhalten. Ferner werden Stentgrafts für die endovaskuläre Behandlung von Aneurysmen, Dissektionen und weiteren spezifischen Läsionen eingesetzt. Der Stentgraft weist dabei üblicherweise eine hohlzylindrische Form mit einer Längserstreckung auf, so dass im implantierten Zustand Blut durch den Hohlraum des Stentgrafts strömen kann.

Damit ein solcher Stentgraft für den Behandlungszeitraum im Blutgefäß eines Patienten jedoch wirksam bleibt, ist es erforderlich, dass der Stentgraft an der zu behandelnden Stelle lagefest ist. Dies wird durch eine hinreichend große Kontaktfläche zwischen der Mantelfäche des Stentgraftes und der Gefäßwand ermöglicht, wobei der Stentgraft mit seiner Mantelfläche innig an der Gefäßwand anliegen muss.

Im Stand der Technik sind daher Stentgrafts bekannt, die einen selbstexpandierenden Stent aufweisen und daher komprimierbar und wieder expandierbar sind. Sie können folglich zumindest zwei Zustände einnehmen, einen komprimierten und einen expandierten Zustand. Zur Implantation wird der Stentgraft zunächst in der radialen Richtung komprimiert, so dass der Durchmesser des hohlzylindrischen Körpers zumindest so weit reduziert wird, dass dieser in das Blutgefäß des Patienten einführbar ist. Um den komprimierten Zustand herzustellen, wird der Stentgraft in eine Hülle eingebracht, die als Hüllkatheter bezeichnet wird. Der Hüllkatheter weist dazu einen Umfang auf, der kleiner ist als der Umfang des Stentgrafts im expandierten Zustand. Im komprimierten Zustand wird der Stentgraft mit dem ihn umgebenden Hüllkatheter in ein Blutgefäß eingeführt und anschließend an der pathologischen Stelle positioniert, wobei die Position des Stentgraftes über Röntgenmarker kontrolliert werden kann. Nachdem die gewünschte Position bzw. Ausrichtung des Stentgraftes erreicht ist, wird der Hüllkatheter zurückgezogen, wodurch der Stentgraft freigesetzt wird und expandieren kann. Der Stentgraft dehnt sich dabei aufgrund der selbstexpandierenden Eigenschaft des Stentes im Blutgefäß auf. Durch die Druckkraft zwischen dem Stentgraft und der Gefäßwand bleibt der Stentgraft an der gewünschten Position lagefest. Somit kann das Blutgefäß offengehalten und der Blutstrom aufrechterhalten werden.

Im Stand der Technik sind bspw. Stentgrafts bekannt, dessen Stent als Drahtstruktur ausgebildet ist, auf die die selbstexpandierende Eigenschaft des Stentes zurückzuführen ist. Die Drahtstruktur besteht normalerweise aus Metall oder Kunststoff und ist ferner von einem Prothesenmaterial umhüllt, das an der Drahtstruktur befestigt ist. Das Prothesenmaterial kann aus textilem Material, bspw. blutdichtem Polyestergewebe, bestehen. Die Bioverträglichkeit solcher Materialien führt dazu, dass der Kontakt zwischen dem expandierten Stentgraft und der Gefäßwand komplikationsfrei ist.

Aus der DE 103 35 948 B3 ist ein Stentgraft mit einem vorstehend genannten Stent bekannt, wobei sich der Stent im expandierten Zustand mit Hilfe eines Fadens wieder komprimieren bzw. zusammenfalten lässt, nachdem der Stentgraft in ein Blutgefäß des Patienten eingebracht wurde. Dadurch wird das Entfernen eines bereits in ein Blutgefäß eingeführten Stentgraftes ermöglicht.

Ein ähnlicher Stentgraft wird in der WO 2011/063972 A1 und in der US 2007/0100427 A1 offenbart, bei dem mit Hilfe einer oder mehrerer Schnüre der Durchmesser des Stentgraftes nach dem Einführen verändert werden kann. Die EP 1 964 532 A2 offenbart einen weiteren Stentgraft, bei dem ein schrittweises Zurückziehen des Hüllkatheters möglich ist. Ein Stentgraft wie im Oberbegriff von Anspruch 1 angegeben ist aus der US 21012/0130475 A1 bekannt.

In allen vorstehend genannten Dokumenten ist der Hüllkatheter als ein separater Hüllschlauch ausgebildet, in den der Körper des Stentgraftes eingeschoben und dabei komprimiert wird. Dies hat jedoch den Nachteil, dass das Komprimieren dadurch erschwert ist, dass die behandelnde Person den Körper des Stentgraftes abschnittsweise zusammenfalten und gleichzeitig in den Hüllkatheter einbringen muss, bis dieser den Körper des Stentgraftes vollständig umhüllt. Ferner ist das Zurückziehen des Hüllkatheters zum Expandieren des hohlzylindrischen Körpers des Stentgraftes mit Reibungskräften zwischen dem Hüllkatheter und der Gefäßwand einerseits und zwischen dem Hüllkatheter und dem Körper des Stentgraftes andererseits behaftet. Dies kann einerseits zu einer Traumatisierung der Gefäßwand und andererseits zu einer Verschiebung oder Verdrehung des Stentgraftes aus seiner zuvor eingestellten optimalen Lage führen. Letzteres bedingt eine aufwändige Nachjustierung der Lage des Stentgraftes im Blutgefäß.

Vor diesem Hintergrund liegt der Erfindung die Aufgabe zugrunde, einen Stentgraft der eingangs genannten Art dahingehend weiterzubilden, dass der Körper auf einfach zu handhabende Weise komprimierbar und in einem Blutgefäß ebenso einfach und weniger reibungsbehaftet freisetzbar und expandierbar ist.

Erfindungsgemäß wird die vorstehend genannte Aufgabe dadurch gelöst, dass der Hüllkatheter als lappenförmiges Komprimierelement ausgebildet ist, das zwei Längsränder aufweist, wobei ein erster Längsrand am Körper befestigt ist, wobei eine Erstreckung des lappenförmigen Komprimierelementes senkrecht zum ersten Längsrand kleiner ist als der Umfang des Körpers im expandierten Zustand, wobei zum Komprimieren des Körpers das lappenförmige Komprimierelement um den Körper herumlegbar und ein freier zweiter Längsrand an am Körper und/oder am lappenförmigen Komprimierelement angeordneten Fixierstellen lösbar festlegbar ist.

Erfindungsgemäß ist der Hüllkatheter als lappenförmiges Komprimierelement ausgebildet und weist zwei Längsränder auf, wobei ein erster Längsrand am Körper befestigt ist. Der Hüllkatheter ist somit im Unterschied zum Stand der Technik am Stentgraft befestigt. Ferner weist das lappenförmige Komprimierelement eine Erstreckung senkrecht zum ersten Längsrand auf, die kleiner ist als der Umfang des Körpers des Stentgraftes im expandierten Zustand. Damit lässt sich der Körper des Stentgraftes mittels des lappenförmigen Komprimierelementes vor dem Einsetzen in ein Blutgefäß komprimieren. Am Körper des Stentgraftes und/oder am lappenförmigen Komprimierelement sind Fixierstellen zum Festlegen des freien Längsrandes des lappenförmigen Komprimierelements angeordnet.

Zum Komprimieren des Körpers des Stentgraftes wird das lappenförmige Komprimierelement um den Körper derart herumgelegt, dass das lappenförmige Komprimierelement den Körper teilweise oder vollständig umhüllt. Dabei wird der Stent radial nach Innen zusammengefaltet und der Querschnitt des Körpers des Stentgraftes verkleinert sich. Der zweite Längsrand wird anschließend an den am Körper und/oder am lappenförmigen Komprimierelement angeordneten Fixierstellen lösbar festgelegt. Somit lässt sich der Körper durch eine Umwicklung komprimieren, was eine vorteilhaft einfachere Handhabung darstellt als das Einführen des Stentgraftes in einen Hüllschlauch. Ein weiterer Vorteil besteht darin, dass der Stentgraft, nachdem er komprimiert worden ist, durch das lappenförmige Komprimierelement eine gleichmäßige Oberfläche besitzt, die das Einsetzen des Stentgraftes in ein Blutgefäß erleichtert.

Nach dem Einsetzen des Stentgraftes in ein Blutgefäß und Positionieren des Stentgraftes an der gewünschten Stelle lässt sich zum Expandieren des Körpers des Stentgraftes der zweite Längsrand an den Fixierstellen lösen. Das lappenförmige Komprimierelement wird jedoch nicht aus dem Blutgefäß herausgezogen, sondern verbleibt am Körper des Stentgraftes im Blutgefäß. Dies wird dadurch ermöglicht, dass das Komprimierelement mit dem ersten Längsrand am Körper befestigt ist, so dass auch nicht die Gefahr besteht, dass das Komprimierelement im Blutgefäß wandert. Dadurch, dass der durch das Komprimierelement gebildete Hüllkatheter nicht aus dem Blutgefäß herausgezogen wird, werden Reibungskräfte zwischen dem Hüllkatheter und der Gefäßwand und zwischen dem Hüllkatheter und dem Körper des Stentgraftes vermieden, wodurch eine Traumatisierung der Gefäßwand und eine Lageveränderung des Stentgraftes mit entsprechender Repositionierung vermieden werden. Ein weiterer Vorteil des erfindungsgemäßen Stentgraftes besteht darin, dass die Operationszeit verkürzt werden kann.

In einer bevorzugten Ausgestaltung erstreckt sich das lappenförmige Komprimierelement im komprimierten Zustand des Körpers senkrecht zum ersten Längsrand zumindest vollumfänglich um den Körper des Stentgraftes herum.

Mit dieser Maßnahme kann vorteilhafterweise der Körper auf seinen minimal möglichen Außendurchmesser komprimiert werden. Der dadurch erreichte komprimierte Zustand des Körpers ist außerdem über den gesamten Umfang des Stentgraftes gleichmäßig, was das Einführen des Stentgraftes ins Blutgefäß und das Positionieren des Stentgraftes im Blutgefäß erleichtert.

In einer weiteren bevorzugten Ausgestaltung weist das lappenförmige Komprimierelement im Wesentlichen eine gleiche Länge wie der Körper auf.

Vorteilhafterweise lässt sich mit dieser Maßnahme der Körper über seine gesamte Länge gleichmäßig komprimieren. Hierdurch wird das Einführen des Stentgraftes in ein Blutgefäß und das Positionieren des Stentgraftes im Blutgefäß zusätzlich erleichtert.

Erfindungsgemäß ist der erste Längsrand des lappenförmigen Komprimierelementes am Prothesenmaterial befestigt.

Diese Maßnahme hat den Vorteil, dass das lappenförmige Komprimierelement besonders einfach am Körper des Stentgraftes fixierbar ist. Das lappenförmige Komprimierelement kann wie das Prothesenmaterial eine textile Struktur aufweisen, sodass sich das lappenförmige Komprimierelement am Prothesenmaterial bspw. durch Nähte einfach befestigen lässt. Eine Befestigung über Nähte hat den Vorteil, dass die Gleichmäßigkeit der Oberfläche des Stentgraftes nicht beeinträchtigt wird, und dass sich das lappenförmige Komprimierelement dennoch nicht unerwünscht vom Körper trennt.

In einer weiteren bevorzugten Ausgestaltung weisen die am Körper und/oder am lappenförmigen Komprimierelement angeordneten Fixierstellen Schlaufen auf, durch die ein Stab zum Festlegen des zweiten Längsrandes des lappenförmigen Komprimierelementes einfädelbar ist.

Vorteilhafterweise wird durch diese Maßnahme ein einfaches Verfahren zum Expandieren des Körpers geschaffen, bei dem keine Reibungskraft zwischen dem Stentgraft und dem Hüllkatheter, sondern lediglich zwischen den Schlaufen und dem Stab entsteht, wobei die dabei auftretende Reibungskraft wesentlich geringer ist als beim Abziehen des Hüllkatheters vom Stentgraft und Herausziehen des Hüllkatheters aus dem Blutgefäß. Der Stab kann eine besonders glatte und damit reibungsarme Oberfläche aufweisen und bspw. aus Edelmetall bestehen und wirkt somit beim Herausziehen nicht traumatisierend auf die Gefäßwand. Somit kann die beim Expandieren des Körpers auftretende Reibungskraft stark reduziert werden und die zum Expandieren benötigte Zeit verkürzt werden. Außerdem wird der Körper beim Herausziehen des Stabes nicht mitbewegt. Ferner lassen sich die Schlaufen einfach, bspw. durch Binden, am Körper und/oder am lappenförmigen Komprimierelement befestigen. Es versteht sich, dass jede Fixierstelle eine oder mehrere Schlaufen aufweisen kann.

In einer weiteren bevorzugten Ausgestaltung weist der zweite Längsrand des lappenförmigen Komprimierelementes einen Hohlsaum auf, in den der Stab einsteckbar ist.

Diese Maßnahme ist vorteilhaft, da der gesamte freie zweite Längsrand durch Einstecken des Stabes, dessen Länge vorzugweise die Länge des ersten Längsrandes übertrifft, gleichmäßig am Körper festlegbar ist. Somit wird die Gleichmäßigkeit des dadurch erzielten komprimierten Zustandes des Körpers des Stentgraftes nach Einstecken des Stabes in den Hohlsaum und Einfädeln des Stabes durch die Schlaufen noch verbessert. Wenn die Schlaufen am oder im Bereich des ersten Längsrandes des Komprimierelementes angeordnet sind, besteht ein weiterer Vorteil dieser Maßnahme darin, dass die beiden Längsränder des lappenförmigen Komprimierelementes direkt miteinander verbindbar sind. Folglich wird im Wesentlichen die gesamte Fläche des lappenförmigen Komprimierelementes zum Komprimieren des Körpers auf seinen kleinst möglichen Durchmesser genutzt.

In einer weiteren bevorzugten Ausgestaltung weist der Hohlsaum mehrere Unterbrechungen auf, wobei die Schlaufen derart angeordnet sind, dass sie sich in Längsrichtung des Körpers auf Höhe der Unterbrechungen befinden.

In dieser Ausgestaltung weist der Hohlsaum einzelne Hohlsaumabschnitte auf, zwischen denen jeweils eine oder mehrere Schlaufen liegen, wenn der Stab durch die Hohlsaumabschnitte und die Schlaufen gefädelt ist. Der Vorteil dieser Maßnahme besteht darin, dass beim Herausziehen des Stabes aus den Schlaufen und den Hohlsaumabschnitten der Körper des Stentgraftes in Längsrichtung gesehen sukzessive, wie in der Art eines Reißverschlusses, freigesetzt und expandiert wird. Damit lässt sich die Positionierung des Stentgraftes im Blutgefäß noch während des allmählichen Freisetzens des Stentgraftes schrittweise optimieren. Außerdem ist der Stab in den Bereichen der Unterbrechungen des Hohlsaums sichtbar, wodurch die Möglichkeit gegeben ist, den Fall eines fehlgeschlagenen Expansionsvorgangs leicht zu erkennen, nämlich wenn ein Abschnitt des Körpers des Stentgraftes trotz Herausziehen des Stabes aus den an diesem Abschnitt des Körpers angeordneten Schlaufen nicht expandiert, sodass Gegenmaßnahmen umgehend ergriffen werden können.

In einer weiteren bevorzugten Ausgestaltung weisen die Unterbrechungen einen gleichmäßigen Abstand voneinander auf.

Mit dieser Maßnahme wird eine gleichmäßige Festlegung des zweiten Längsrandes des lappenförmigen Komprimierelementes ermöglicht.

In einer weiteren bevorzugten Ausgestaltung besteht das lappenförmige Komprimierelement aus resorbierbarem Material.

Der Vorteil dieser Maßnahme besteht darin, dass sich das Komprimierelement nach dem Einsetzen und Expandieren des Stentgraftes, wenn es seine Funktion erfüllt hat, abbauen kann. Es versteht sich, dass das Material des Komprimierelementes biokompatibel ist. Das Komprimierelement kann auch aus einem textilen Gewebe bestehen, unabhängig, ob resorbierbar oder nicht.

In einer weiteren bevorzugten Ausgestaltung weist das lappenförmige Komprimierelement eine radiale Faltenstruktur auf.

Im expandierten Zustand des Körpers wirkt eine der Expansion zugrunde liegende Dehnungskraft des Stentgraftes im Wesentlichen auf die durch das lappenförmige Komprimierelement aufgespannte Fläche, wobei durch die radiale Faltenstruktur der Stentgraft im Blutgefäß noch lagefester ist. Vorteilhafterweise erhöht diese Maßnahme die Lagefestigkeit des erfindungsgemäßen Stentgraftes im expandierten Zustand.

In einer weiteren bevorzugten Ausgestaltung weist das lappenförmige Komprimierelement eine reibungsarme, vorzugsweise anformbare, weiter vorzugsweise atraumatische Oberfläche auf.

Der Vorteil dieser Maßnahme besteht darin, dass das Komprimierelement sich mit geringer Reibung an der Gefäßwand einführen lässt und gegenüber der Gefäßwand des Patienten nicht traumatisierend wirkt. Außerdem kann sich der Hüllkatheter aufgrund seiner Anformbarkeit an die innere Form bzw. an den Verlauf des Blutgefäßes leicht anpassen und an die Gefäßwand gut anschmiegen.

In einer weiteren bevorzugten Ausgestaltung besteht das lappenförmige Komprimierelement aus einem textilen Material.

Der Vorteil dieser Maßnahme besteht darin, dass das Komprimierelement (Lappen) im Vergleich zu dem Hüllkatheter wesentlich flexibel ist. Dadurch ist es möglich auch bei stark gewundenen Gefäßanatomien das Stentgraft Systems zum Absetzpunkt vorzuschieben. Hierbei wird unter "textilem Material" jedes textile Flächengebilde verstanden, insbesondere solche aus Fasern, wie Geflechte, Gewebe, Netze und Gewirke. Die Fasern stellen dabei Endlos-Fasern dar, die die Verspinnbarkeit als Eigenschaft aufweisen, und, wie zuvor erwähnt, verflochten, gewebt, vernetzt oder gewirkt werden können.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Fig. 1: einen erfindungsgemäßen Stentgraft im vollständig expandierten Zustand in einer Seitenansicht;
- Fig. 2: den Stentgraft in Fig. 1 im vollständig expandierten Zustand ausschnittsweise in perspektivischer Anicht;
- Fig. 3: den Stentgraft in Fig. 1 im komprimierten Zustand in Seitenansicht;
- Fig. 4: einen Ausschnitt des Stentgrafts in Fig. 3 im komprimierten Zustand in gegenüber Fig. 3 vergrößertem Maßstab;
- Fig. 5: den Stentgraft in einem Zustand ausgehend von dem Zustand in Fig. 3 zu Beginn seiner Freisetzung;
- Fig. 6: den Stentgraft in einem weiteren Zustand bei fortgesetzter Freisetzung;
- Fig. 7: den Stentgraft in einem noch weiteren Zustand bei weiter fortgesetzter Freisetzung; und
- Fig. 8: den Stentgraft im vollständig expandierten Zustand nach vollständiger Freisetzung.

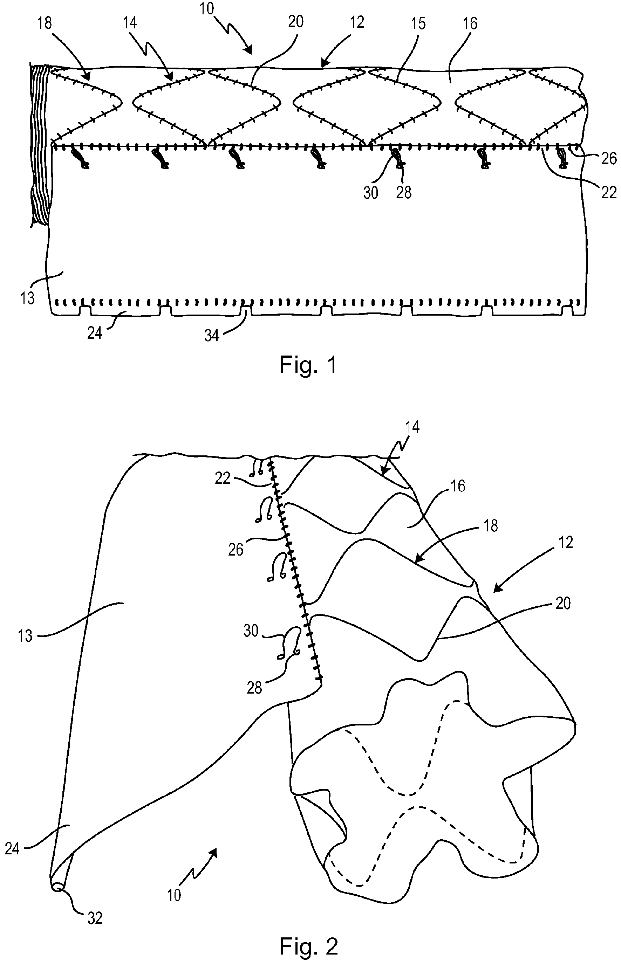

In Fig. 1 und 2 ist ein Ausführungsbeispiel eines mit dem allgemeinen Bezugszeichen 10 versehenen Stentgraftes gezeigt, der einen hohlzylindrischen Körper 12 und einen Hüllkatheter aufweist, der als ein lappenförmiges Komprimierelement 13 ausgebildet ist. Der Körper 12 weist einen selbstexpandierenden Stent 14 und ein Prothesenmaterial 16 auf. Der Stent 14 weist in der Längsrichtung des hohlzylindrischen Körpers 12 hintereinander folgende Ringsegmente 18 aus mäanderförmig umlaufenden Stützen 20 auf. Das Prothesenmaterial, beispielsweise eine textile Struktur, ist an den Ringsegmenten 18 durch Nähte 15 befestigt, wobei die Nähte in allen weiteren Figuren nicht gezeigt sind.

Der Körper 12 ist, wie der perspektivischen Ansicht in Fig. 2 zu entnehmen ist, hohlzylindrisch. Das lappenförmige Komprimierelement 13 weist einen am Körper 12 befestigten ersten Längsrand 22 und einen freien zweiten Längsrand 24 auf, wobei der erste Längsrand 22 am Prothesenmaterial 16 durch Nähte 26 befestigt ist. Das lappenförmige Komprimierelement 13 weist senkrecht zu beiden Längsrändern 22, 24 eine Erstreckung auf, die dem Umfang des Körpers 12 im komprimierten Zustand im Wesentlichen entspricht. Das lappenförmige Komprimierelement 13 weist in Längsrichtung. d.h. parallel zu den Längsrändern, im Wesentlichen eine gleiche Länge wie der Körper 12 auf.

Am Körper 12 und/oder lappenförmigen Komprimierelement 13, hier am lappenförmigen Komprimierelement 13, sind Fixierstellen 28 angeordnet, die im Bereich des ersten Längsrandes 22 des lappenförmigen Komprimierelementes 13 befestigte Schlaufen 30 aufweisen. Am zweiten Längsrand 24 ist ferner ein Hohlsaum 32 ausgebildet, der Unterbrechungen 34 aufweist. Die Unterbrechungen 34 des Hohlsaums 32 sind derart angeordnet, dass sie sich in Längsrichtung des Körpers 12 auf Höhe der Schlaufen 30 befinden. Ferner sind die Unterbrechungen 34 mit einem gleichmäßigen Abstand voneinander entfernt angeordnet.

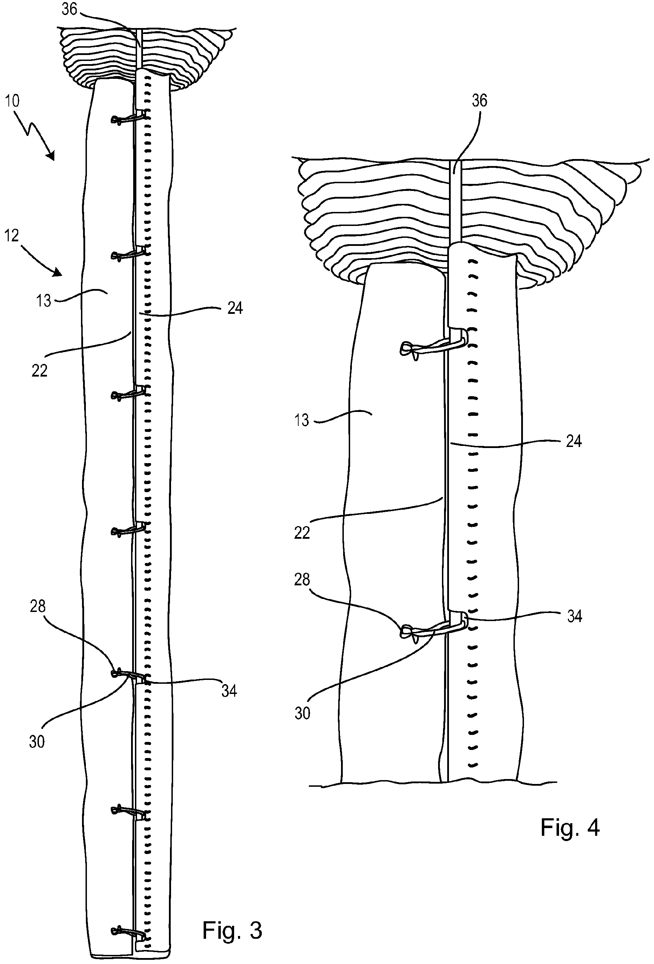

Vor dem Einbringen des Stentgraftes 10 in ein Blutgefäß (nicht gezeigt) eines Patienten (nicht gezeigt) wird der Stentgraft 10 zunächst komprimiert. Die Ausgangssituation für das Komprimieren zeigen Fig. 1 und Fig. 2. Beim Komprimieren wird das lappenförmige Komprimierelement 13 derart um den Körper 12 herumgelegt, dass beide Längsränder 22, 24 des lappenförmigen Komprimierelementes 13 im Wesentlichen aufeinander liegen. Dabei wird der Körper 12 radial auf einen Umfang komprimiert, der kleiner ist als der Umfang des Körpers 12 im expandierten Zustand, wie aus Fig. 3 und Fig. 4 zu entnehmen ist.

Um den komprimierten Zustand des Stentgraft 10 vor und während des Einsetzens des Stentgrafts in ein Blutgefäß aufrechtzuerhalten, wird ein Stab 36 in den Hohlsaum 32 an einem Längsende des Komprimierelements 13 eingeführt und durch den Hohlsaum 32 hindurchgeführt. Dabei werden die Schlaufen 30, die sich nun in den Bereichen der Unterbrechungen 34 befinden, auf den Stab 36 ebenfalls aufgefädelt. Wenn wie in Fig. 3 dargestellt alle Schlaufen 30 vom Stab 36 aufgefädelt sind, ist der Längsrand 24 festgelegt und der Körper 12 behält nun den komprimierten Zustand bei.

In diesem gleichmäßig komprimierten Zustand kann der Stentgraft 10 in ein Blutgefäß des Patienten eingeführt und an einer zu behandelnden Stelle im Blutgefäß positioniert werden. Anschließend wird der Körper 12 freigesetzt und in den expandierten Zustand überführt, indem der Stab 36 aus dem Hohlsaum 32 und den Schlaufen 30 wieder herausgezogen wird. Die Expansion des Körpers 12 erfolgt dabei selbsttätig aufgrund der selbsexpandierenden Eigenschaft des Stents 14.

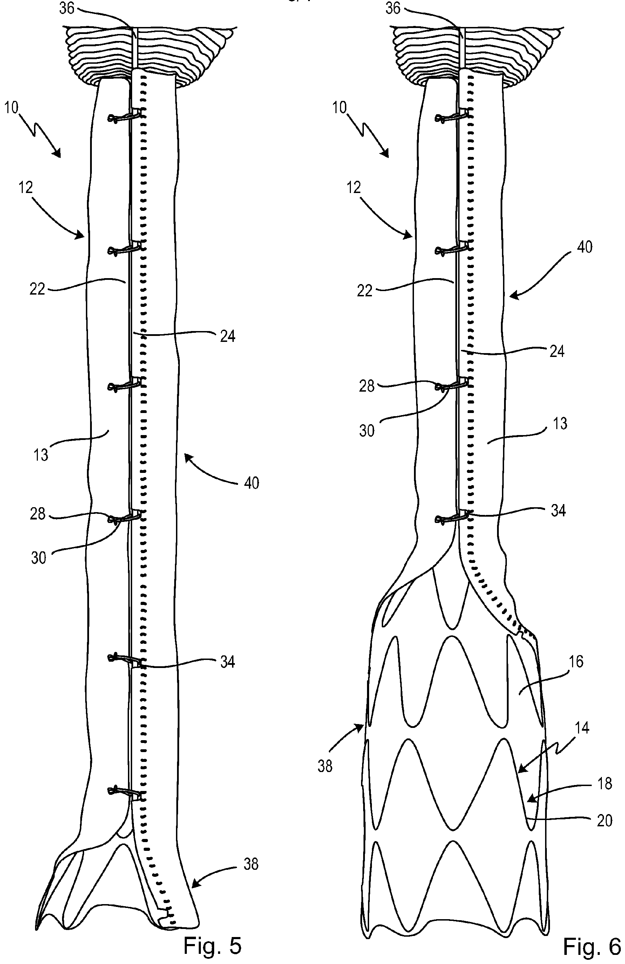

In den Figuren 5 bis 8 ist der erfindungsgemäße Stentgraft 10 in mehreren verschiedenen Stadien während des sukzessiven Freisetzens und Expandierens gezeigt. Zum Expandieren des Stentgraftes 10 wird der Stab 36 in Längsrichtung allmählich aus dem Hohlsaum 32 und den Schlaufen 32 herausgezogen. Sobald sich eine erste der Schlaufen 30 vom Stab 36 abtrennt, löst sich an dieser Stelle der Längsrand 24 des lappenförmigen Komprimierelementes 13 entlang eines ersten Abschnitts 38 des Körpers 12, der sich zwischen dieser ersten der Schlaufen 30 und der nächsten der Schlaufen 30 befindet. Der erste Abschnitt 38 des Körpers 12 dehnt sich aufgrund der Selbstexpansionsfähigkeit des Stentes 14 auf und geht in den expandierten Zustand über. Ein restlicher Abschnitt 40 des Körpers bleibt zunächst weiterhin gleichmäßig komprimiert. Durch weiteres Herausziehen des Stabes 36 werden nach und nach die übrigen Abbschnitte des Körpers 12 freigesetzt und expandiert.

Darüber hinaus ist für den Stentgraft 10 möglich, dessen Positionierung während des Expansionsvorgangs des Körpers 12 zu optimieren. Insbesondere kann man die Positionierung des Stentgraftes 10 schrittweise optimieren, indem der Stab 32 schrittweise aus dem Hohlsaum 32 herausgezogen wird.

In einem der in Fig. 5 bis 7 dargestellten Zustände des Stentgraftes 10, in denen der Stentgraft 10 noch teilweise komprimiert und bereits teilweise expandiert ist, insbesondere in einem Zustand gemäß Fig. 5 und 6, lässt sich die Position bzw. Lage des Stentgrafts 10 durch Schieben bzw. Drehen optimieren.

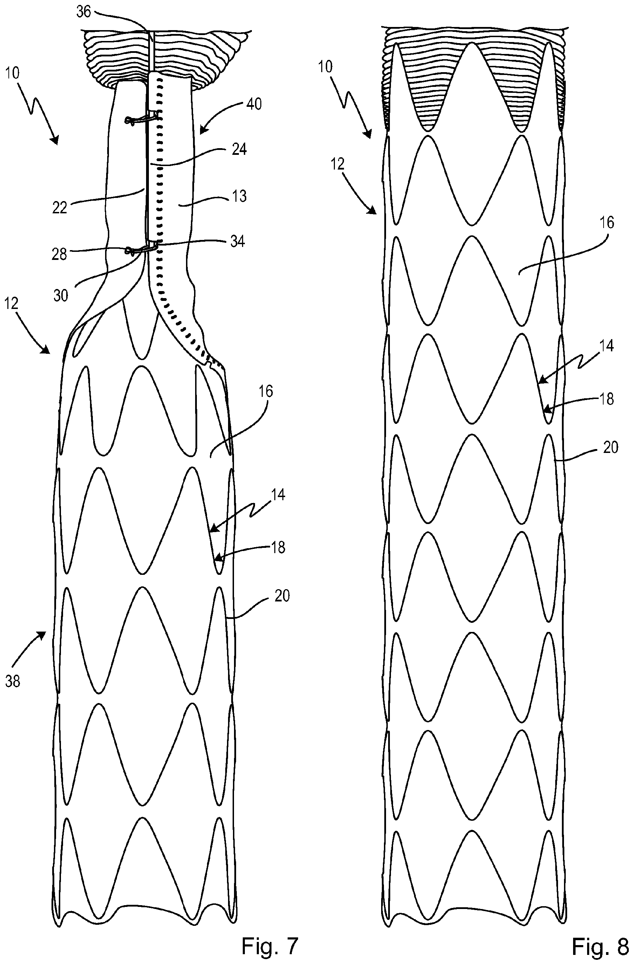

Nachdem die Positionierung des Stentgraftes 10 gegebenenfalls optimiert wurde und keine weiteren Korrekturen vorgenommen werden sollen, kann der Stab 36 komplett aus dem Hohlsaum 32 herausgezogen werden, um den erfindungsgemäßen Stentgraft 10 vollständig expandieren zu lassen. Der vollständig expandierte Zustand des Stentgraftes 10 ist in Fig. 8 gezeigt. Das Komprimierelement 13 (in Fig. 8 nicht zu sehen) verbleibt am Körper 12, d.h. es ist mit seinem ersten Längsrand 22 am Körper 12 weiterhin festgelegt.

Das lappenförmige Komprimierelement 13 kann aus textilem Gewebe gefertigt werden, wobei die Form, Schmiegsamkeit und die Oberflächenbeschaffenheit des lappenförmigen Komprimierelementes 13 optimal an die durch die Form und den Verlauf der Blutgefäße gestellten Anforderungen angepasst werden können. Insbesondere kann das lappenförmige Komprimierelement 13 eine radiale Faltenstruktur aufweisen, die die Lagefestigkeit des erfindungsgemäßen Stentgraftes 10 im Blutgefäß noch erhöht. Unter Verwendung von resorbierbarem Material lässt sich das lappenförmige Komprimierelement 13 so herstellen, dass es sich nach einer gewissen Zeit im Blutgefäß bioverträglich abbaut. Unabhängig davon ist das Komprimierelement bevorzugt aus einem bioverträglichen Material gefertigt, so dass eine möglichst komplikationsfreie Behandlung mit dem erfindungsgemäßen Stentgraft 10 möglich ist.

## Patentansprüche

1. Stentgraft (10), mit einem hohlzylindrischen Körper (12) und einem Hüllkatheter, wobei der Körper (12) zumindest einen selbstexpandierenden Stent (14) und ein Prothesenmaterial (16) aufweist, wobei der Körper (12) in einem komprimierten Zustand einen Umfang aufweist, der kleiner ist als ein Umfang des Körpers (12) in einem expandierten Zustand, wobei der Hüllkatheter als lappenförmiges Komprimierelement (13) ausgebildet ist, das zwei Längsränder (22, 24) aufweist, **dadurch gekennzeichnet, dass** ein erster Längsrand (22) am Prothesenmaterial (16) des Körpers (12) befestigt ist, wobei eine Erstreckung des lappenförmigen Komprimierelementes (13) senkrecht zum ersten Längsrand (22) kleiner ist als der Umfang des Körpers (12) im expandierten Zustand, wobei zum Komprimieren des Körpers (12) das lappenförmige Komprimierelement (13 ) um den Körper (12) herumlegbar und ein freier zweiter Längsrand (22) an am Körper (12) und/oder am lappenförmigen Komprimierelement (13) angeordneten Fixierstellen (28) lösbar festlegbar ist.

2. Stentgraft nach Anspruch 1, **dadurch gekennzeichnet, dass** sich das lappenförmige Komprimierelement (13) im komprimierten Zustand des Körpers (12) senkrecht zum ersten Längsrand (22) zumindest vollumfänglich um den Körper (12) herum erstreckt.

3. Stentgraft nach Anspruch 1 oder 2, **dadurch gekennzeichnet, dass** das lappenförmige Komprimierelement (13) im Wesentlichen eine gleiche Länge wie der Körper (12) aufweist.

4. Stentgraft nach einem der Ansprüche 1 bis 3, **dadurch gekennzeichnet, dass** die am Körper (12) und/oder am lappenförmigen Komprimierelement (13) angeordneten Fixierstellen (28) Schlaufen (30) aufweisen, durch die ein Stab (36) zum Festlegen des zweiten Längsrandes (24) des lappenförmigen Komprimierelementes (13) einfädelbar ist.

5. Stentgraft nach Anspruch 4, **dadurch gekennzeichnet, dass** der zweite Längsrand (24) des lappenförmigen Komprimierelementes (13) einen Hohlsaum (32) aufweist, in den der Stab (36) einsteckbar ist.

6. Stentgraft nach Anspruch 5, **dadurch gekennzeichnet, dass** der Hohlsaum (32) mehrere Unterbrechungen (34) aufweist, wobei die Schlaufen (30) derart angeordnet sind, dass sie sich in Längsrichtung des Körpers (12) auf Höhe der Unterbrechungen (34) befinden.

7. Stentgraft nach Anspruch 6, **dadurch gekennzeichnet, dass** die Unterbrechungen (34) einen gleichmäßigen Abstand voneinander aufweisen.

8. Stentgraft nach einem der Ansprüche 1 bis 7, **dadurch gekennzeichnet, dass** das lappenförmige Komprimierelement (13) aus textilem Gewebe oder resorbierbarem Material besteht.

9. Stentgraft nach einem der Ansprüche 1 bis 8 **dadurch gekennzeichnet, dass** das lappenförmige Komprimierelement (13) eine radiale Faltenstruktur aufweist.

10. Stentgraft nach einem der Ansprüche 1 bis 9, **dadurch gekennzeichnet,** dassdas lappenförmige Komprimierelement (13) eine reibungsarme, vorzugsweise anformbare, weiter vorzugsweise atraumatische Oberfläche aufweist.

## Claims

1. A stent graft (10) with a hollow cylindrical body (12) and a sleeve catheter, wherein the body (12) has at least one self-expanding stent (14) and a prosthetic material (16), wherein the body (12), in a compressed state, has a circumference that is smaller than a circumference of the body (12) in an expanded state, wherein the sleeve catheter is designed as a flap-shaped compression element (13) having two longitudinal edges (22, 24), **characterized in that** a first longitudinal edge (22) is secured on the prosthetic material of the body (12), wherein an extent of the flap-shaped compression element (13) perpendicular to the first longitudinal edge (22) is smaller than the circumference of the body (12) in the expanded state, wherein, for the purpose of compressing the body (12), the flap-shaped compression element (13) can be placed around the body (12) and a free, second longitudinal edge (22) is releasably attachable to fixation points (28) arranged on the body (12) and/or on the flap-shaped compression element (13).

2. The stent graft as claimed in claim 1, **characterized in that** the flap-shaped compression element (13), in the compressed state of the body (12), extends perpendicularly with respect to the first longitudinal edge (22) and at least fully circumferentially about the body (12).

3. The stent graft as claimed in claim 1 or 2, **characterized in that** the flap-shaped compression element (13) has substantially the same length as the body (12).

4. The stent graft as claimed in one of claims 1 through 3, **characterized in that** the fixation points (28) arranged on the body (12) and/or on the flap-shaped compression element (13) have loops (30) through which a rod (36) can be threaded for securing the second longitudinal edge (24) of the flap-shaped compression element (13).

5. The stent graft as claimed in claim 4, **characterized in that** the second longitudinal edge (24) of the flap-shaped compression element (13) has a hemstitch (32) into which the rod (36) is insertable.

6. The stent graft as claimed in claim 5, **characterized in that** the hemstitch (32) has several interruptions (34), wherein the loops (30) are arranged in such a way that, in the longitudinal direction of the body (12), they are located at the level of the interruptions (34).

7. The stent graft as claimed in claim 6, **characterized in that** the interruptions (34) are at a uniform distance from one another.

8. The stent graft as claimed in one of claims 1 through 7, **characterized in that** the flap-shaped compression element (13) is made of textile fabric or resorbable material.

9. The stent graft as claimed in one of claims 1 through 8, **characterized in that** the flap-shaped compression element (13) has a radial fold structure.

10. The stent graft as claimed in one of claims 1 through 9, **characterized in that** the flap-shaped compression element (13) has a low-friction, preferably formable, more preferably atraumatic surface.

## Revendications

1. Greffe d'endoprothèse (10), comprenant un corps cylindrique creux (12) et un cathéter enveloppant, le corps (12) présentant au moins une endoprothèse auto-expansible (14) et un matériau prothétique (16), le corps (12) présentant une circonférence dans un état comprimé qui est inférieure à une circonférence du corps (12) dans un état expansé, le cathéter enveloppant étant configuré sous forme d'élément de compression en forme de languette (13), qui présente deux bords longitudinaux (22, 24), **caractérisée en ce qu'**un premier bord longitudinal (22) est fixé au matériau prothétique (16) du corps (12), une étendue de l'élément de compression en forme de languette (13) perpendiculairement au premier bord longitudinal (22) étant inférieure à la circonférence du corps (12) à l'état expansé ; pour comprimer le corps (12), l'élément de compression en forme de languette (13) pouvant être placé autour du corps (12) et un deuxième bord longitudinal libre (22) pouvant être immobilisé de manière amovible en des points de fixation (28) agencés sur le corps (12) et/ou sur l'élément de compression en forme de languette (13).

2. Greffe d'endoprothèse selon la revendication 1, **caractérisée en ce que** l'élément de compression en forme de languette (13) s'étend au moins entièrement autour du corps (12) à l'état comprimé du corps (12) perpendiculairement au premier bord longitudinal (22).

3. Greffe d'endoprothèse selon la revendication 1 ou 2, **caractérisée en ce que** l'élément de compression en forme de languette (13) présente essentiellement une même longueur que le corps (12).

4. Greffe d'endoprothèse selon l'une quelconque des revendications 1 à 3, **caractérisée en ce que** les points de fixation (28) agencés sur le corps (12) et/ou sur l'élément de compression en forme de languette (13) présentent des boucles (30), à travers lesquelles peut être enfilée une tige (36) pour fixer le deuxième bord longitudinal (24) de l'élément de compression en forme de languette (13).

5. Greffe d'endoprothèse selon la revendication 4, **caractérisée en ce que** le deuxième bord longitudinal (24) de l'élément de compression en forme de languette (13) présente un ourlet creux (32), dans lequel la tige (36) peut être insérée.

6. Greffe d'endoprothèse selon la revendication 5, **caractérisée en ce que** l'ourlet creux (32) présente plusieurs interruptions (34), les boucles (30) étant agencées de telle sorte qu'elles se trouvent à hauteur des interruptions (34) dans la direction longitudinale du corps (12).

7. Greffe d'endoprothèse selon la revendication 6, **caractérisée en ce que** les interruptions (34) présentent un espacement uniforme les unes des autres.

8. Greffe d'endoprothèse selon l'une quelconque des revendications 1 à 7, **caractérisée en ce que** l'élément de compression en forme de languette (13) est constitué de tissu textile ou de matériau résorbable.

9. Greffe d'endoprothèse selon l'une quelconque des revendications 1 à 8, **caractérisée en ce que** l'élément de compression en forme de languette (13) présente une structure plissée radiale.

10. Greffe d'endoprothèse selon l'une quelconque des revendications 1 à 9, **caractérisée en ce que** l'élément de compression en forme de languette (13) présente une surface à faible frottement, de préférence malléable, de manière davantage préférée atraumatique.
